# EUROPEAN PATENT SPECIFICATION

(11) **EP 1 829 893 B1**
(45) Date of publication and mention of the grant of the patent: **21.09.2011**
(21) Application number: 05811052.9
(22) Date of filing: 16.11.2005
(51) Int. Cl.: C07K 14/55

(54) **IMMUNOTHERAPEUTIC FORMULATIONS WITH INTERLEUKIN-2-NEUTRALISING CAPACITY**
IMMUNTHERAPEUTISCHE FORMULIERUNGEN MIT DER FÄHIGKEIT, INTERLEUKIN-2 ZU NEUTRALISIEREN
FORMULATIONS INMUNOTHERAPEUTIQUES A CAPACITE DE NEUTRALISATION DE L'INTERLEUKINE-2

(30) Priority: 16.11.2004 CU 20040261
(43) Date of publication of application: 05.09.2007
(60) Divisional application: 09164216.5
(73) Proprietor: Centro de Inmunologia Molecular, Ciudad de la Habana 12100 (CU)
(72) Inventor: MONTERO CASIMIRO, José Enrique, La Lisa, Ciudad de La Habana 17 100 (CU); ALONSO SARDUY, Liván Bladimir, Habana Vieja, Ciudad de La Habana 10200 (CU); PEREZ RODRIGUEZ, Rolando, Ciudad de La Habana 10500 (CU); LAGE DAVILA, Agustín Bienvenido, Ciudad de La Habana 10600 (CU)
(74) Representative: van Loon, C.J.J.
(86) International application number: PCT/CU2005/000009
(87) International publication number: WO 2006/053508

(56) References cited:
- EP-A2- 0 474 313
- WO-A2-02/087304
- WO-A2-03/045997
- US-A- 5 614 185
- US-A- 5 830 452
- US-A- 6 060 068
- CHEN T T ET AL: "IDIOTYPE-CYTOKINE FUSION PROTEINS AS CANCER VACCINES RELATIVE EFFICACY OF IL-2, IL-4, AND GRANULOCYTE-MACROPHAGE COLONY-STIMULATING FACTOR" JOURNAL OF IMMUNOLOGY, AMERICAN ASSOCIATION OF IMMUNOLOGISTS, US, vol. 153, no. 10, 1 January 1994 (1994-01-01), pages 4775-4787, XP008062345 ISSN: 0022-1767

### SEQUENCE LISTING

<110> Centro de Inmunologia Molecular
<120> Immunotherapeutic formulations to generate autoantibodies capable to avoid the binding of interleukin-2 to its receptor. Their use in the treatment of cancer.
<130> P81062EP00
<140> EP 05811052.9
   <141> 2007-06-18
<150> PCT/CU2005/000009
   <151> 2005-11-16
<150> CU 261-2004
   <151> 2004-11-16
<160> 2
<170> PatentIn version 3.5
<210> 1
   <211> 18
   <212> PRT
   <213> Homo sapiens
<400> 1
   Asn Pro Lys Leu Thr Arg Met Leu Thr Phe Lys Phe Tyr Met Pro Lys
   1               5                   10                  15
   Lys Ala
<210> 2
   <211> 21
   <212> PRT
   <213> Homo sapiens
<400> 2
   Thr Ile Val Glu Phe Leu Asn Arg Trp Ile Thr Phe Cys Gln Ser Ile
   1               5                   10                  15
   Ile Ser Thr Leu Thr
               20

## Description

### Field of the Invention.

The present invention is related to pharmaceutical formulations able to increase the immune response against the Interleukin-2 (IL-2) and rising autoantibodies, which blockade the binding to the receptor, and which are useful in the treatment of tumors.

### Description of the Prior Art.

The discovery of the capacity of the Immune System and specifically of T cells to recognize tumor-antigens is one of the fundamental pillars for the development of strategies for the manipulation of the immune system with the aim to treat patients with cancer.

Consequently, to the development of methodologies to recover specific T cells infiltrating the tumor stroma, known as Tumor-Infiltrating Lymphocyte (TIL), or originated from peripheral blood of non-treated individuals or after the of therapeutic cancer vaccines, the main effort has been directed to the stimulation of these cells to increase their anti-tumor effector capacity *in vivo.*

Therefore, the main strategies have been directed to enhance their specific cytotoxic activity against a variety of Tumors Associated Antigens (TAA). The main therapeutic approach has been focused on the *in vitro* use of interleukin-2 (IL-2) to activate and expand TIL from tumor-bearing individuals who are re-infused then with these cells (Rosenberg, S. A. et al (1986) Science 233, 1318-1321; Kawakami, Y. et al. (1994) Proc. Natl. Acad. Sci. USA 91, 6458-6462; Kawakami, Y. et al. (1994) Proc. Natl. Acad. Sci. USA 91, 3551-3519). However, such interventions have limited therapeutic results despite the demonstrated stimulation of the cellular immune response obtained *in vitro.*

This has led to evaluate therapeutic modalities based on the use of active specific immunization protocols with therapeutic cancer vaccines, designing vaccine vectors that contain tumor-antigens associated to IL-2 in order to facilitate the induction of the effective cellular immune response *in vivo,* but these approaches have rendered poor results (Rosenberg, S. A., et al. (1998) Nat. Med. 4, 321-327).

Currently, the main clinical strategies have shift to the development of a modality based on the adoptive transference of reactive T cells from a patient to it own tumor-antigens. These cells are stimulated and expanded *in vitro* using anti-CD3 Monoclonal Antibodies (mAb) and lL-2, and after re-infusion into the blood stream IL-2 parenteral administration is provided. This approach constitutes one of main therapeutical interventions conceived for the treatment of cancer patients, although the therapeutic results continue being discreet (Dudley, M. E., et al. (2002) Science 298, 850-854; Rosenberg, S. A. et al. (2004) Proc Natl Acad Sci U S A. 101 Suppl 2, 14639-45).

The rational design of all these therapeutic strategies is based on the use of Interleukin-2 as an essential molecule in the cellular activation of the anti-tumor immune response (US 6,060,068 and US 5,830,452).

The background for IL-2 function in immunity is based on experiments performed *in vitro.* From its discovery, IL-2 was recognized by the capacity to stimulate T cell proliferation (so, the IL-2 acronym is T Cells Growth Factor). Further demonstration of T cell proliferation and function *in vitro* could be inhibited using an anti-IL-2 or an anti-IL-2 receptor confirmed this notion (Smith, KA. Immunol Rev 51:337-357, 1980).

Recently, it has been experimentally demonstrated that human tumors can reduce the response of the Immune System through the generation of T cells with a suppressor capacity of the anti-tumor immunity. These cells have been characterized in animal models and patients displaying different differentiation markers, although their relevance differ from the experimental model (Bach, J.F. (2003) Nat Rev Immunol 3, 189-198; Chakraborty, N.G., et al. (2004) Hum Immunol 65, 794-802; Markus, Y.M.y Sykes, M. (2004) J Clin Oncol 22, 1136-11151).

The Cluster of Differentiation 25 (CD25) constitutes the alpha chain of the IL-2 receptor. Besides, the structure of the receptor for this cytokine comprises the beta (CD122) and gamma (CD132) chains. They are constitutively expressed in resting T lymphocytes and the activation of these cells induces the synthesis of the alpha chain, the formation of the high affinity heterotrimeric receptor and the IL-2 secretion. The CD25 is expressed constitutively in 5 - 10 % of CD4+ T iymphocytes and in less than 1 % of peripheral CD8+ T lymphocytes. These cells are anergic and display a suppressor activity *in vitro* (Shevach, E.M. (2002) Nat Rev Immunol 2, 389-400).

It has been recently demonstrated that passive administration of anti-CD25 mAb induces antitumor response in some experimental tumors, although others are refractory to such therapy (Onizuka, S. et al. (1999) Cancer Res 59, 3128-3133).

The capacity of the Immune System to induce a response against self-molecules is limited, specifically to soluble molecules such as growth factors. Nevertheless, active immunization with these factors conjugated to a carrier protein and emulsified in adjuvants promotes the induction of the immune response against these molecules (U.S. 5.984.018). The specific autoantibodies generated against the autologous or heterologous molecules inhibit the binding to their receptor, blocking the mechanisms of proliferation triggered by this binbind

From those results, it is accepted as the state-of-the-art that an anti-tumor response depends on the IL-2 presence. Consequently, we decided to characterize *in vivo* the impact on the tumor evolution of anti-IL-2 autoantibodies induced by active immunization with IL-2 conjugated to a carrier molecule in an adjuvant. Surprisingly, the induction of blocking autoantibodies of IL-2 binding to its receptor promotes the reduction of the tumor growth, even in tumors that are resistant to the antitumor effect induced by the passive administration of anti-CD25 mAb. Additionally, the presence of such autoantibodies does not affect the immune response to cancer vaccines in treated subjects.

### Detailed description of the invention:

The present invention is related to therapeutic formulations that have an effect in the treatment of tumors, for which the role of the Immune System of the subject is important. Particularly, the present invention comprises the generation of immunotherapeutic formulations able to raise autoantibodies which blockades the binding of the interleukin-2 to its receptor and inhibits the growth of tumors.

The object of the present invention is a therapeutic formulation to elicit an immune response against IL-2, for use in the treatment of cancer patients, comprising human recombinant IL-2 coupled to a carrier protein by genetically or chemical conjugation, wherein the carrier protein is P64k from *Neisseria meningitidis,* and an appropriate adjuvant. Particularly, the adjuvant is selected from the group comprising aluminum hydroxide and Montanide ISA 51.

In an embodiment of the invention the therapeutic formulation comprises the IL-2 conjugated to P64k by chemical conjugation. In another embodiment of the invention the formulation comprises a fusion protein between IL-2 and P64K.

Also part of the invention is a therapeutic formulation to elicit an immune response against IL-2, further comprising a monoclonal antibody anti-IL-2, a cancer vaccine based on specific tumour antigens or growth factors, and/or a monoclonal antibody anti-CD25.

The invention further provides a therapeutic formulation according to the invention in combination with a cancer vaccine based on specific tumour antigens or growth factors for use in the treatment of cancer. Said cancer vaccine preferably comprises EGF.

The invention further provides a therapeutic formulation according to the invention in combination with a specific monoclonal antibody anti-CD25, for use in the treatment of cancer. The invention also provides a therapeutic formulation to elicit an immune response against IL-2 according to the invention, further comprising a specific anti-CD25 monoclonal antibody.

The invention further provides a therapeutic formulation according to the invention for the manufacture of a medicament to elicit an immune response against IL-2 able to inhibit the growth of tumours in cancer patients.

The term "combination" refers to a physical combination (i.e in mixture form) as well as to the association of two different and separated organizations (example in form of reagents) and when they are used in combination to treat a patient. Thus, pharmaceutical combinations are those useful combinations in the treatment schedule that involves the administration of two compounds, combined by physical association or administered in independent doses to the same patent in the course of the therapy.

The term "cancer vaccines" refers about to a useful agent in the active immunotherapy so that it causes in the subject submissive treatment by inducing an immune response that recognizes the antigen used in the vaccine and that can be measured.

### 1.- Obtaining the immunogenic composition.

The vaccine composition of the present invention contains as active principle the human recombinant Interleukin-2 (hIL-2r) conjugated to a carrier protein, a protein derived from the external membrane complex of the Neisseria meningitidis P64k (0474313 EP A2 and U.S. 5.286.484). Additionally this vaccine composition contains an appropriate adjuvant. The vaccine composition of the present invention uses preferably the Montanide ISA 51 as an adjuvant.

The conjugation between hIL-2r and the carrier protein can be by chemical conjugation or construction of a fusion protein obtained by techniques of genetic engineering.

### - Obtaining a vaccine composition that contains hIL-2r conjugated to the P64k protein by a chemical conjugation.

To obtain a protein conjugation between the hIL-2r and the P64k protein, both components are mixed in a variable proportion from 20:1 to 5:1 (mole of hIL-2r by mole of the P64k protein), preferably 10:1 (mole of hIL-2r by mole of the P64k protein). Glutaraldehyde is added to this mixture to a final concentration that ranks between 0,02 and 0,1 %, preferably 0,5% and incubated at room temperature (RT) from 1-5 hours. Finally, it is dialyzed extensively in a Phosphate-Buffered Saline (PBS) solution.

The conjugation reaction is verified by a 10% SDS polyacrylamide gel electrophoresis (Laemmli UK (1970) Nature 277,680-685).

### - Obtaining a vaccine composition that contains a fusion protein between hIL-2r and the P64k protein.

The gene that codifies for the human IL-2 (447 bp) is amplified by polymerase chain reaction (PCR) using specific primers. The resulting DNA fragment is digested and attached in a specific binding site of an expression vector in which is cloned the gene that codifies for the carrier protein, so that the resulting protein includes a copy or more of both molecules. It can be used any expression vector for mammalian cells as well as for bacteria or yeast. The vector also may include six histidines in the N-terminal end of the carrier protein. The resulting plasmid is verified by restriction analysis in agarose gel electrophoresis, analysis of the DNA sequence using the enzyme Sequenace 2,0 (Amersham-USB), and finally, analysis of the production of the fusion protein in any expression strain of *Eschericia coli,* through the "Western Blotting" technique, using a specific anti-hIL-2 monoclonal antibody. In order to obtain the protein the bacterial walls are broken using a method of strong rupture, and then the protein is purified by a combination of differential precipitation methods with ammonium sulphate and chromatographic methods. Finally, the protein is filtered in sterile conditions and conserved at -20°C or lyophilized and conserved at 4°C until its later use.

### - Obtaining vaccine compositions that contain peptides derived from hIL-2r chemically conjugated to the P64k protein or coupled as a fusion protein.

Peptides derived from the amino acids sequence of the IL-2 obtained by chemical synthesis can be coupled by chemical conjugation to the P64k protein, as it is described in U.S. 5,984,018. Alternatively, the fusion protein of derived peptide from the hIL-2r and the P64k protein are obtained essentially by the same method described in the previous section. Examples may include peptides from the following regions:

| | | |
|---|---|---|
| 1) | Peptide | N³³-A⁵⁰ |
| | Number of amino acids: | 18 |
| | Sequence: | NPKLTRMLTFKFYMPKKA |
| | | |
| 2) | Peptide | T¹¹³-T¹³³ |
| | Number of amino acids: | 21 |
| | Sequence: | TIVEFLNRWITFCQSIISTLT |

### - Electrophoresis of chemical conjugate hIL-2r-P64k.

It can be made by a 10% SDS polyacrylamide get electrophoresis (Laemmli U.K. (1970) Nature 277,680-685). It is applied 15 µg of sample per well and it is stained with Coomassie.

### 2.- Characterization of the effect produced by the vaccine composition that contains the hIL-2r and the protein P64k. Pre-clinical studies.

### - Immunogenicity of the vaccine composition.

In order to study in animals the immunogenicity of the vaccine preparation of the present invention, female BALB/c mice aged 8-12 weeks of age and 18 - 20 g of weight were used. During the experiment, mice were housed under standard conditions for feeding and manipulation established under the Standard Operation Procedures (SOPs), according to the Good Practices of Care of and Use of the Experimental Animals.

Different immunization schedules can be followed:
- Schedule A, four doses of 4-µg equivalents of hIL-2r conjugated in the hIL-2r-P64k/Montanide ISA 51 vaccine in 0.1 mL are administered by intramuscular route every two weeks, alternating the immunization sites in the limbs.
- Schedule B four doses of 10-µg equivalents of hIL-2r conjugated in the hIL-2r-P64k/Montanide ISA 51 vaccine in 0.1 mL are administered by intramuscular route. The first two doses are administered simultaneously in independent immunization sites in two limbs and two weeks later, two doses are administered in the other two limbs.

### - Evaluation of the antibody response against hIL-2r induced by the hIL-2r-P64k/Montanide ISA 51 vaccine.

Autoantibodies titers can be detected in serum by one of the methods available in the state-of-the-art for measuring blocking antibodies or evaluating the specific immune response in peripheral blood, measuring specific antibodies or B cells.

Anti-hIL-2r antibodies induced by the vaccine hIL-2r-P64k/Montanide ISA 51 can be evaluated by an indirect ELISA (enzyme-linked immunosorbent assay) with the sera from immunized animals as it is described:
96 wells flat-bottomed micro titer plates (Costar, High Binding, USA) are covered with 50 µL/Twell of hIL-2r, prepared to a concentration of 10 µg/mL in a carbonate-bicarbonate 0.1M, pH 9.6 solution. The plates are incubated overnight at 4°C, and washed 2 times with 200 µL/well of PBS containing 0,05 % of Tween 20 (PBS-Tween 20). After incubating 1 hour at 37°C with 100 µL/well containing 1 % of bovine serum albumin (PBS-BSA 1 %), plates are washed with 200 µL/well of PBS-Tween 20 and then added 50 µL/well of the serum samples at different dilutions in PBS-BSA 1%. After 1 hour incubation at 37°C plates are washed with PBS-Tween 20 and 50 µL/well of a sheep anti-mouse serum conjugated to alkaline phosphatase (Jackson Immunoresearch Laboratories) diluted 1/5000 in PBS-BSA is added and incubated 1 hour at 37°C. Plates are washed and 50 µL/well of the substrate solution is added, consisting in 1 µg/mL of p-nitrophenylphosphate (Sigma) in a diethanolamine buffer solution, pH 9.8. After incubating the plates 30 minutes at room temperature (RT), the absorbance of the product of the reaction is measured in an ELISA reader (Organon Teknika, Austria) at 405 nm.

### - Evaluation of the anti-hEGF antibody response induced by a EGF based vaccine

Autoantibodies titers can be detected in serum by one of the methods available in the state-of-the-art for measuring blocking antibodies or evaluating the specific immune response in peripheral blood, measuring specific antibodies or B cells.

Anti- hEGF antibodies induced by an EGF based vaccine can be evaluated by an indirect ELISA (enzyme-linked immunosorbent assay) with the sera from immunized animals, as it is described in Gonzalez, G., et al. (2002) Vaccine Research 5, 233-244.

### - Effect on CTLL-2 cell line proliferation after incubation with sera from animals vaccinated with hIL-2-P64k/Montanide ISA 51 or a specific Monoclonal Antibody (mAb) to hIL-2r.

IL-2-dependent T-cell line CTLL-2 is kept in RPMI-1640 medium containing 1U/mL of hIL-2h under continuous proliferation. CTLL-2 culture is performed in 75 cm² cell culture flask containing 25 mL of RPMI-1640 with 8 - 20 % of Fetal Calf Serum (FCS) with 0.5x10⁵ -10⁸ cells/mL cells in suspension. The cells are used two days after in vitro expansion.

To perform the assay, cells are extracted from the in vitro culture and washed not less than four times with RPMI-1640 or PBS. In 96 wells flat bottom culture plates (Costar, High Binding, USA), 5x10³ cells are seeded. These cells are treated with sera dilutions from immunized animal with the hlL-2-P64klMontanide ISA 51 vaccine with an ELISA titer to IL-2 of 1:10000 or with an anti-IL-2 specific mAb and 1U/mL of hlL-2r is added. Culture is performed for 24 hours in a humid atmosphere at 37°C in a 5 % CO₂ incubator. Proliferation was determined by pulsing with [³H]thymidine (1 µCi/well) for the final 18 to 24 hours of culture. Thymidine incorporation is measured in a liquid scintillation counter. All manipulations are made in sterile conditions.

### 3- Anti-tumor effect of the hlL-2-P64k/Montanide ISA 51 vaccine. Pre-clinical studies.

In order to study in animals the anti-tumor effect of the vaccine preparation of the present invention, female BALB/c mice aged 8 - 12 weeks of age and 18 - 20 g of weight are used. Animals can be immunized with the hIL-2-P64k/Montanide ISA 51 vaccine according to the schedules A and B previously detailed, and one week later inoculated with a tumor cell line, as the breast carcinoma F3II at 5x10⁴ cells/animal by the subcutaneous route orthotopically. Mice bearing palpable tumors are scored as positive and tumor growth is measured using a calliper, the longest surface length (a) and its perpendicular width (b) were defined, and tumor size reported as a x b. Tumor are checked periodically.

Surprising and unexpectedly the authors of the present invention have found that after binding neutralization of the IL-2 to it receptor in malignant tumor-bearing subjects, the immune response against this tumor is enhanced, inducing a reduction of the tumor size.

The state of the previous technique indicated that such action would cause an inhibition of the response of the immune system of the individual to the tumor.

The authors have found that this effect on the tumor growth is obtained when the circulating IL-2 levels are substantially reduced, when the subject is under active therapy with the vaccine composition of the present invention like when this reduction is obtained under passive therapy with anti-IL-2 monoclonal antibodies.

For that reason, the present invention results of undeniable advantages for the treatment of patients suffering from malignant tumors and provides a vaccination method with IL-2 that is effective, simple and much less annoying and aggressive for the patients than the conventional treatments used in these cases.

The following examples include the experimental details, illustrating the immunological effectiveness of the vaccine compositions subject of the invention.

### EXAMPLES:

The human recombinant IL-2 (hIL-2r) used in the vaccine composition of the present invention was commercially available (U.S. 5.614.185).

The protein P64k from *Neisseria Meningitidis* used in the vaccine was obtained by the recombinant DNA technology described in EP 0474313 A2 and U.S. 5,286,484.

### EXAMPLE 1: Antibody response induced by the hIL-2r-P64k/Montanide ISA 51 vaccine.

In order to promote the immunogenicity against the human Interleukin-2 it was chemically conjugated to the carrier protein P64k protein from the *Neisseria Meningitidis.*

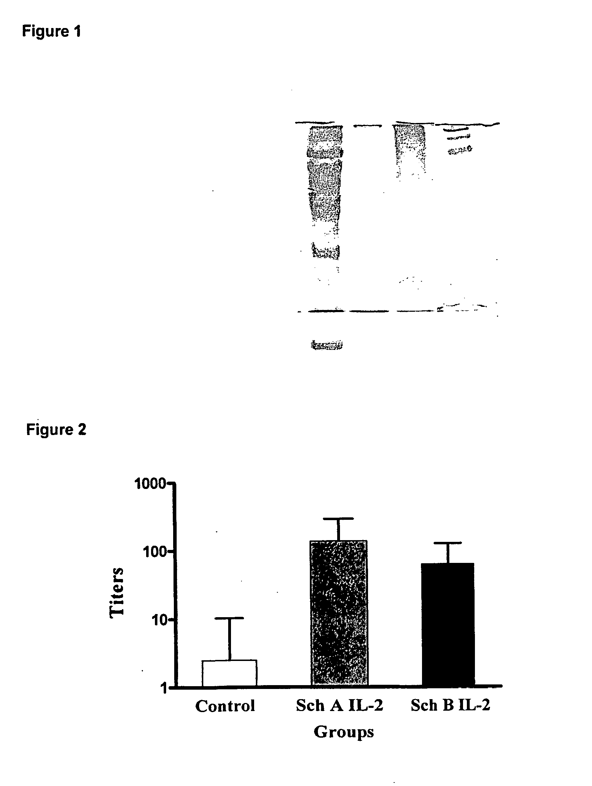

The chemical conjugation can be made by the method of Glutaraldehyde (U.S. 5.984.018). By a 10% SDS polyacrylamide gel electrophoresis the efficiency of the conjugation is verified, applying samples of each component separately (hIL-2r, P64k) compared against the chemical conjugate hIL-2r-P64k and a standard pattern of molecular weight. It is possible to verify that a conjugate was obtained due to existence of a continuous band in the lane where the hIL-2r-P64k was applied (Figure 1).

In order to evaluate the antibody response to hIL-2r induced by the hIL-2r-P64k/Montanide ISA 51 vaccine, BALB/c mice were immunized with the schedules A and B. A hyperimmune serum from an animal that respond to the hIL-2r-P64k/Montanide ISA 51 vaccine was used as positive control, and a pre-immune serum as negative control. It was defined as the antibody titer of the immunized animals, the greater dilution to which the optical density of serums was greater than the average value of the optical density of the pre-immune serum plus five times the standard deviation. In order to define the titers in the control animals the same previous criterion was used unless PBS-BSA 1% instead of an pre-immune serum as negative control were used.

Antibody response was induced reaching titers of 1:100 to1:50000. This protocol of immunization last about 52 days, reason why was necessary to modify it to obtain a similar or better antibody titers in a shorter time period, so we used the schedule B and obtained similar results, as it is shown in Figure 2.

### EXAMPLE 2: Protiferation test of CTLL-2 cell line treated with sera from immunized animals with the hIL-2-P64k/Montanide. ISA 51 vaccine.

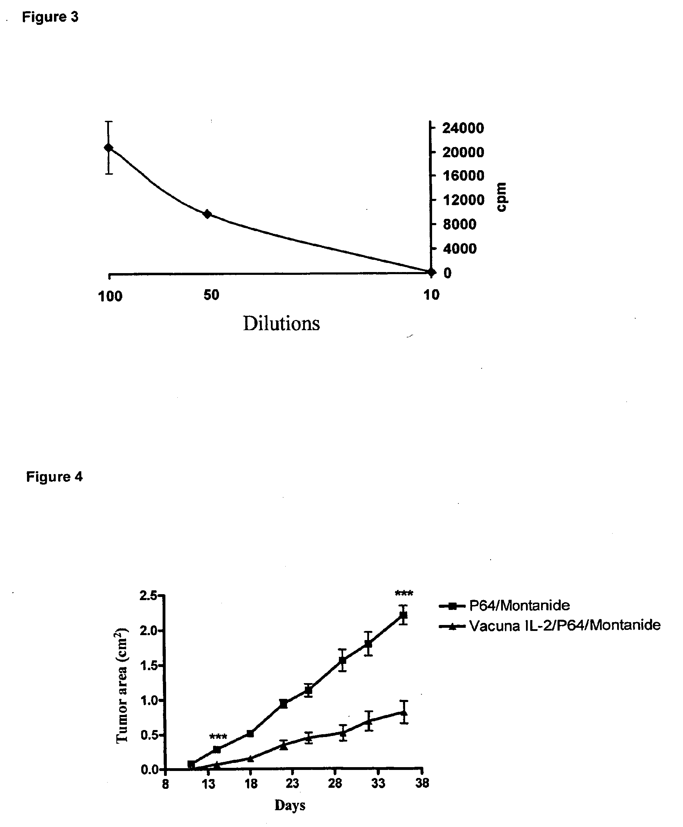

The in vitro IL-2 binding capacity of serum antibodies generated in animals immunized with the hIL-2r-P64k/Montanide ISA 51 vaccine was evaluated by culturing them with the IL-2-dependent T-cett line CTLL-2. CTLL-2 cells were seeded in culture plates in the presence of IL-2 to let them growth and different sera dilutions from animal with about 1:10000 antibody titres were added. It was observed a direct correlation between the serum concentration and the inhibition of the CTLL-2 cell line proliferation (Figure 3). It demonstrates the IL-2 neutralization capacity in vitro of the serum from vaccinated animals.

### EXAMPLE 3: Anti-tumor experiments in animals treated with the hIL-2-P64k/Montanide ISA 51 vaccine.

Animals were immunized with the hIL-2r-P64k/Montanide ISA 51 vaccine following the schedule B. One week later animals were challenged with 5x10⁴ F3II tumor cells. The tumor growth kinetics was slower in hIL-2r-P64k/Montanide ISA 51 immunized animals compared with the group control, immunized with PBS-P64k/Montanide ISA 51 (Figure 4a). There was observed statistical significant differences in the tumor size between these two groups.

### EXAMPLE 4: Proliferation test of CTLL-2 cell line treated with an anti-IL-2 specific mAb.

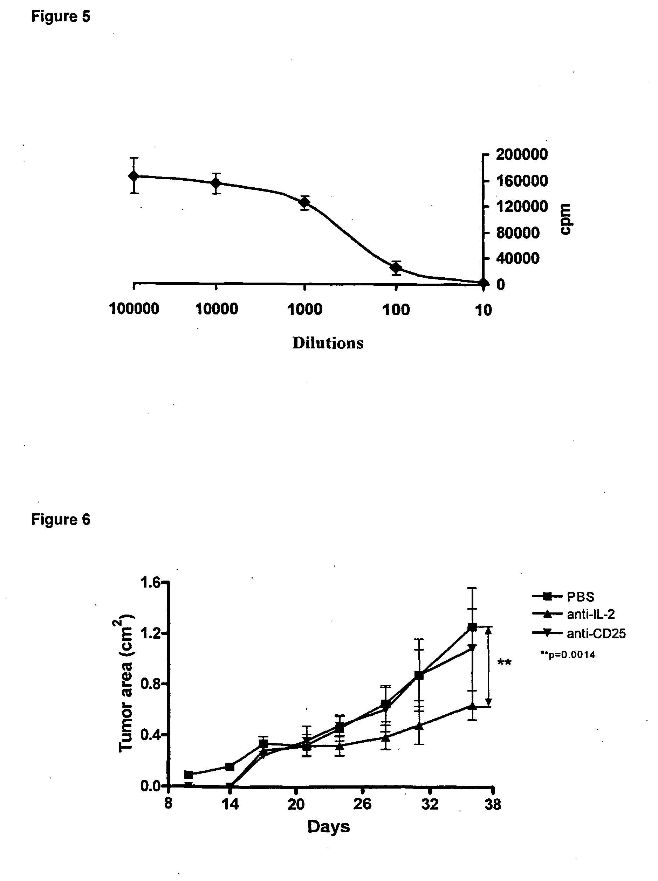

The in vitro IL-2 binding capacity of the specific antibodies against hIL-2r produced by the S4B6 (ATCC # HB-8794) hybridoma were evaluated by culturing them with the IL-2-dependent T-cell line CTLL-2. CTLL-2 cells were seeded in culture plates in the presence of IL-2 to let them growth and different antibody dilutions were added. It was observed a direct correlation between the antibody concentration and the inhibition of the CTLL-2 cell line proliferation (Figure 5). It was found that a 1:100 antibody dilution is able to inhibit in more than 80% of the CTLL-2 cell line proliferation, demonstrating the IL-2 neutralization capacity in vitro of the monoclonal antibody.

### EXAMPLE 5: Anti-tumor effect of the anti-IL-2 mAb and an anti-CD25 mAb (ATCC-PC61). F3II experimental model (breast carcinoma).

Mice were treated with a daily dose of 1 mg of the specific monoclonal antibody (anti-IL-2 mAb or anti-CD25 mAb) during five consecutives days. Two days later mice were challenged with 5x10⁴ cells/mouse of the experimental breast carcinoma F3II by a subcutaneous orthotropic injection. The longest surface length (a) and its perpendicular width (b) were measured periodically. The tumor size in the control group (treated with PBS) was higher than in the group treated with the anti-IL-2 mAb (Figure 6). The tumor size between the two experimental groups was statistically different. However, the tumor size in the anti-CD25 mAb (ATCC - PC61) treated animals was similar to the control group. This result shows that IL-2 neutralizing antibodies have a potent anti-tumor effect even in tumors where removal of CD25 regulatory cells has no effect.

### EXAMPLE 6: Anti-tumor effect of the anti-IL-2 mAb and an anti-CD25 mAb (ATCC-PC61). EL4 experimental model (lymplioma).

C57BL/6 animals were challenged subcutaneously 5x10⁴ cells/mouse of the EL4 lymphoma. Animals were treated intravenously with one dose of 1mg of an anti-CD25 mAb (PC61) two days before the tumor challenge or with a daily dose of 1 mg of an anti-IL-2 mAb (S4B6) during five consecutive days starting six days before the tumor inoculation. Other groups were co-administered with the anti-CD25 and anti-IL-2 mAbs based on a similar schedule previously describes.

Tumor growth of each group of mice was recorded was measured in the perpendicular dimensions twice weekly after tumor challenge. Statistical differences for EL4 tumor are *p0.0232, **p=0.0039, ***p<0.001.

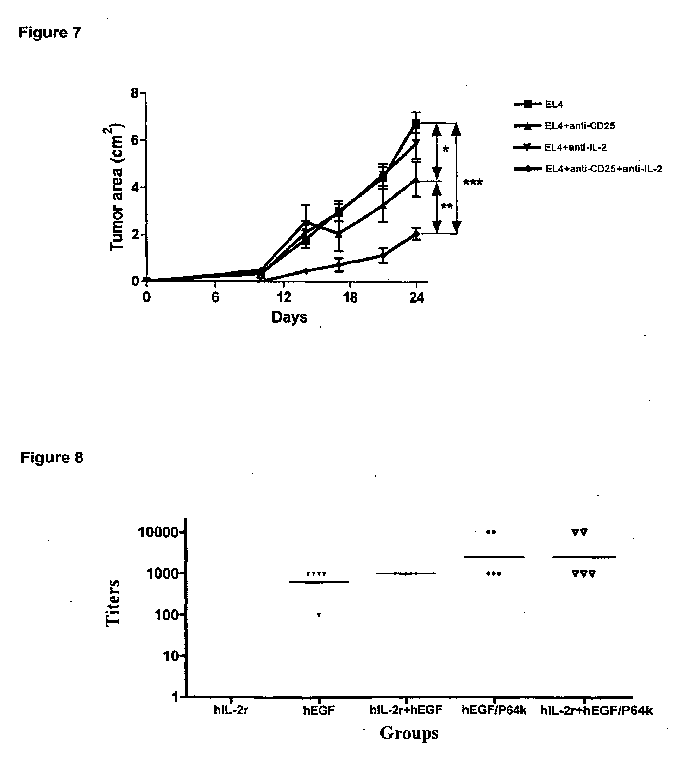

Figure 7 shows thats the combination of the two monoclonal antibodies had a strong effect on the tumor growth.

### EXAMPLE 7: The induction of autoantibodies to anti-IL-2 does not affect the response to EGF cancer vaccine.

To evaluate if the antibody response against hEGF induced by the vaccine hEGF-P64k/Montanide ISA 51, were affected by the previous vaccination with hlL-2r, which entails to the induction of autoantibodies, BALB/c animals were immunized with the vaccine prepared hIL-2r-P64k/Montanide ISA 51 according to the schedule A and 9 days later immunized with vaccine prepared hEGF-P64k/Montanide ISA 51 or with hEGF/Montanide ISA 51. In all the experiments where it was immunized with the vaccine of hEGF, 4 µg equivalent of hEGF conjugated in the vaccine prepared in a volume of 0.1mL, administered by intramuscular route. An hyperimmune serum of respondent animals to the vaccine hEGF-P64k/Montanide ISA 51 was used as positive control and negative control an pre-immune serum. It was defined as titers of antibodies in the immunized animals, the greater dilution to which the optical density of serums was greater than the average value of the optical density of the pre-immune serum more five times the standard deviation. In order to define the titers in the animal controls the same previous criterion was used unless PBS-BSA 1% instead of an pre-immune serum as negative control were used. It was obtained that the titers of antibodies against EGF induced by the vaccination with EGF/Montanide ISA 51 or hEGF-P64k/Montanide ISA 51 are not affected by the induction of autoantibodies against hIL-2r as is shown in Figure 8

### EXAMPLE 8: Neutralization of Interleukin-2 in vivo restore the cytolytic activity evaluated in lymph node cells of tumor-bearing hosts

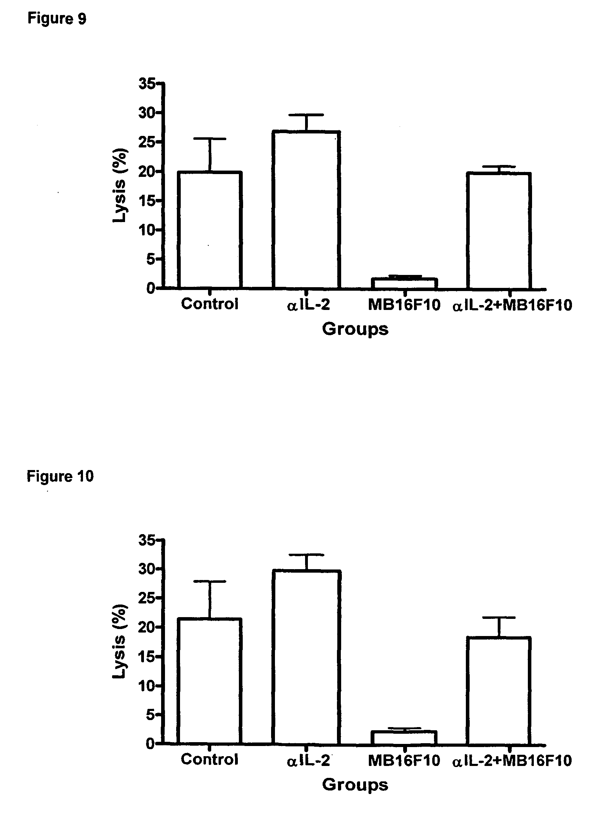

The IL-2 neutralization effect on the immunoresponse to a nominal antigen in a tumor-bearing host was evaluated. Female C57BL/6J mice (H-2b) were maintained under standard housing conditions. For all experiments, mice between the ages of 6 and 12 wk were used. Ovalbumin (OVA) and peptides: OVA grade VII (Sigma, St. Louis, MO) was the model proteinAg used throughout these experiments. The dominant peptide OVA₂₇₅₋₂₆₄ (SIINFEKL) were used at a purity > 90%. Proliferation assay: C57BL/6 mice were challenged with 10⁴ cells of MB16F10 tumor o PBS by subcutaneous route in the left flank. Tumor diameters measure periodically. Three weeks later these mice were immunized s.c. on day 0 with 1 mg of OVA with 100 µg/mouse of Polyinosinic:polycytidylic acid [poly I:C] (PIC) (Sigma, St. Louis, MO) and the subsequent 2 days only PIC was administered. Simultaneously, animals were treated with the specific monoclonal antibody to hIL-2r (αIL-2) or PBS five consecutives days. In vivo cytolytic activity was determined using spleen cells from naïve mice differentially labelled with the fluorescent dye CFSE (Molecular probes, Paisley, UK). The cells labelled with CFSEhigh were used as targets and pulsed with SIINFEKL (1 µM); 90 min at 37°C, 5 % CO₂), whereas the cells labelled with CFSElow were left unpulsed to serve as the internal control. Peptide-pulsed target cells were extensively washed to remove free peptide and then co-injected intravenously in a 1:1 ratio to previously immunized mice. Sixteen hours later, lymph nodes and spleens were removed and the total events corresponding to both fluorescent intensities (CFSElow and CFSEhigh) were determined by flow cytometry. The ratio between the percentages of uncoated vs SIINFEKL-coated (CFSE^{tnt}/CFSE^{high}) was calculated to obtain a numerical value of cytotoxicity. The cytolytic activity of lymph node cells to the OVA peptide evaluated in vivo in C57BL/6 mice immunized with OVA plus PIC rendered a maximun response. Administration of a daily dose during 5 consecutives days of 1 mg of anti-IL-2 monoclonal antibody by intravenous injection does not affect the cytolytic response in these animals. C57BL/6 mice challenged with MB16F10 tumor become immunosuppressed with a reduced cytolytic activity to OVA. However, the administration in vivo of a monoclonal antibody with an IL-2 neutralization capacity restored the immune response of lymph node cells (Figure 9).

### EXAMPLE 9: Neutralisation of interleukin-2 in vivo restore the cytolytic activity evaluated in spleen cells of tumor-bearing hosts

The IL-2 neutralisation effect on the immunoresponse to a nominal antigen in a tumor-bearing host was evaluated. C57BL/6 mice were immunized with OVA plus PIC and the cytolytic activity of spleen cells to an OVA peptide was evaluated in vivo obtaining a maximum response. Administration of a daily dose during 5 consecutives days of 1 mg of anti-IL-2 monoclonal antibody by intravenous injection does not affect the cytolytic response in these animals.

C57BL/6 mice challenged with MB16F10 tumor become immunosuppressed with a reduced cytolytic activity to OVA. However, the administration in vivo of a monoclonal antibody with an IL-2 neutralization capacity restore the immune response of spleen cells (Figure 10).

### EXAMPLE 10: Count of blood cells in animals immunized with prepared vaccine

### hIL-2- P64k/Montanide ISA 51.

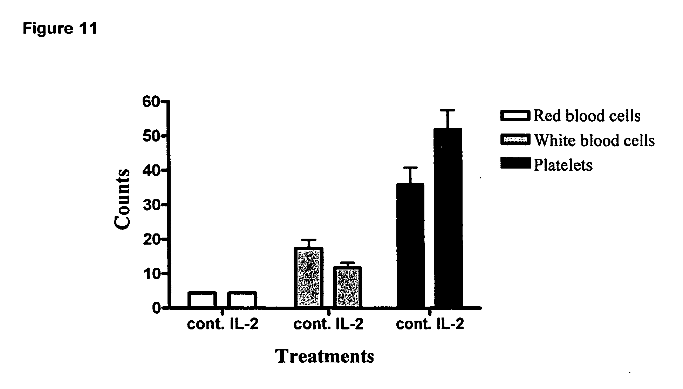

To evaluate if the immunization with vaccine prepared hIL-2r-P64k/Montanide ISA 51 induced changes in the number of cells of the blood red blood cells, white blood cells and platelets, were counted in animals immunized with vaccine prepared (according to schedule A) or in animals without vaccinating, at 100 days after the first immunization. As shown in Figure 11, one did not obtain differences in the cellular counts between the analysed groups.

### Brief description of the figures:

**Figure 1**- Electrophoresis of chemical conjugated hIL-2r-P64k. The bands of left to right correspond to the P64k, hIL-2r, hIL-2r-P64k and a standard pattern of molecular weight, respectively.
**Figure 2****-** Titers of antibodies against hIL-2r induced by the vaccination hIL-2r-P64k/Montanide ISA 51 using explained schedules of immunization both previously. The *y* axis represents the geometric average of the titer of antibodies against IL-2. The x axis the groups that correspond to animals immunized with:
   Control: P64k/Montanide ISA 51.
   Sch A IL-2: hIL-2r-P64k/Montanide ISA 51 according to schedule A
   Sch B IL-2: hIL-2r-P64k/Montanide ISA 51 according to schedule B.
**Figure 3****-** Proliferation of the CTLL-2 line in the presence of hIL-2r and different dilutions from animal serum immunized with the vaccine hIL-2r-P64k/Montanide ISA 51 and that had titles of the order of 1:1000 up to 1:50 000.
**Figure 4****-** Tumor growth of animals vaccinated with the vaccine of hIL-2r-P64k/Montanide ISA 51 or with P64k/Montanide ISA 51 (group control) and later challenged with F3II tumor. The *y* axis and represents the tumor area defined as diameter greater xsmatter diameter selected perpendicularly.
**Figure 5****-** Proliferation of the CTLL-2 line in the presence of hIL-2r and different dilutions from antibodies against IL-2.
**Figure 6****-** Tumor growth of animals treated with the anti-IL-2 mAb, with the anti-CD25 mAb or PBS (group control) and challenged with the F3II tumor cell line. The *y* axis represents the tumor area defined as diameter greater x smaller diameter selected perpendicularly.
**Figure 7****-** Tumor growth of animals treated with the αCD25 mAb, with the anti-CD25 mAb or PBS (group control) and later challenged with lymphoma EL4. The *y* axis represents the tumor area defined as diameter greater x smaller diameter selected perpendicularly.
**Figure 8****-** Antibody titers to EGF induced by the vaccination with hIL-2r-P64k/Montanide ISA 51 and hEGF-P64k/Montanide ISA 51. The *y* axis represents the geometric mean of the titers of antibodies to EGF. The x axis represents the groups immunized with:
   hIL-2r. hIL-2r-P64k/Montanide ISA 51.
   hEGF: hEGF/Montanide ISA 51
   hIL-2r+hEGF: hIL-2r-P64k/Montanide + hEGF/Montanide.
   hEGF/P64k: hIL-2r-P64k/Montanide + hEGF/Montanide.
   hIL-2r+hEGF/P64k: hIL-2r-P64k/Montanide + hEGF/P64k/Montanide.
**Figure 9** **-** Induced antibodies to Interleukin-2 restore the cytolytic activity evaluated in lymph node cells of tumor-bearing hosts.
   All mice were immunized subcutaneously on day 0 with 1 mg of OVA plus 100 µg per mouse of PIC and the subsecuents 2 days only PIC was administered. In vivo cytolytic activity was determined using spleen cells from naïve mice differentially labelled with the fluorescent dye CFSE. The cells labelled with CFSEhigh were used as targets and pulsed with SIINFEKL, whereas the cells labelled with CFSElow were leaved unpulsed to serve as the internal control, then co-injected intravenously in a 1:1 ratio to previously immunized mice. Cytolytic activity was evaluated in lymph node cells.
   Control -animals treated with PBS, αIL-2 -animals treated with an IL-2 neutralizing monoclonal antibody, MB16F10 -animals similar to control but challenged with MB16F10, αIL-2+MB16F10 -MB16F10 tumor-bearing mice treated with IL-2 neutralizing monoclonal antibody.
**Figure 10** - Induced antibodies to Interleukin-2 restore the cytolytic activity evaluated in spleen cells of tumor-bearing hosts.
   All mice were immunized subcutaneously on day 0 with 1 mg of OVA plus 100 µg per mouse of PIC and the subsecuents 2 days only PIC was administered. In vivo cytolytic activity was determined using spleen cells from naïve mice differentially labelled with the fluorescent dye CFSE. The cells labelled with CFSEhigh were used as targets and pulsed with SIINFEKL, whereas the cells labelled with CFSElow were leaved unpulsed to serve as the internal control, then co-injected intravenously in a 1:1 ratio to previously immunized mice. Cytolytic activity was evaluated in spleen cells.
   Control -animals treated with PBS, αIL-2 -animals treated with an IL-2 neutralizing monoclonal antibody, MB16F10 -animals similar to control but challenged with MB16F10, αIL-2+MB16F10 -MB16F10 tumor-bearing mice treated with IL-2 neutralizing monoclonal antibody.
**Figure 11**- Blood Cell Count (leukocytes (white blood cells), erythrocytes (red blood cells) and platelets) in animals immunized with the hIL-2r-P64k/Montanide ISA 51 vaccine compare to a control (P64k/Montanide ISA 51).

## Claims

1. A therapeutic formulation to elicit an immune response against IL-2, useful for use in the treatment of cancer patients, comprising human recombinant IL-2 coupled to a carrier protein by genetically or chemical conjugation, wherein the carrier protein is P64k from *Neisseria meningitidis,* and an appropriate adjuvant.

2. A therapeutic formulation to elicit an immune response against IL-2, according to claim 1, wherein the adjuvant is selected from the group comprising aluminium hydroxide and Montanide ISA 51.

3. A therapeutic formulation to elicit an immune response against IL-2, according to claim 1, wherein the adjuvant is Montanide ISA 51.

4. A therapeutic formulation to elicit an immune response against IL-2, according to claim 1, comprising a chemical conjugation between IL-2 and P64k.

5. A therapeutic formulation to elicit an immune response against IL-2, according to claim 1, comprising a fusion protein between IL-2 and P64k.

6. A therapeutic formulation to elicit an immune response against IL-2, according to claim 1, further comprising a monoclonal antibody anti-IL-2, a cancer vaccine base on specific tumour antigens or growth factors, and/or a monoclonal antibody anti-CD25.

7. A therapeutic formulation according to claim 1 in combination with a cancer vaccine based on specific tumour antigens or growth factors for use in the treatment of cancer.

8. A therapeutic formulation to elicit an immune response against IL-2, according to claim 7, wherein the cancer vaccine comprises EGF.

9. A therapeutic formulation to elicit an immune response against IL-2, according to claim 1 in combination with a specific monoclonal antibody anti-CD25, for use in the treatment of cancer.

10. A therapeutic formulation to elicit an immune response against IL-2, according to claim 1, further comprising a specific anti-CD25 monoclonal antibody.

11. A therapeutic formulation according to claims 1 a 7, for the manufacture of a medicament to elicit an immune response against IL-2 able to inhibit the growth of tumours in cancer patients.

## Patentansprüche

1. Therapeutische Formulierung, um eine Immunreaktion auf IL-2 auszulösen, zur Verwendung bei der Behandlung von Krebspatienten, umfassend: rekombinantes Human-IL-2, das durch genetische oder chemische Konjugation an ein Trägerprotein gekoppelt ist, wobei das Trägerprotein P64k von Neisseria meningitidis ist, und ein geeignetes Adjuvans.

2. Therapeutische Formulierung nach Anspruch 1, wobei
das Adjuvans ausgewählt ist aus der Gruppe, die Aluminiumhydroxid und Montanide ISA 51 umfasst.

3. Therapeutische Formulierung nach Anspruch 1, wobei das Adjuvans Montanide ISA 51 ist.

4. Therapeutische Formulierung nach Anspruch 1, umfassend:
eine chemische Konjugation zwischen IL-2 und P64k.

5. Therapeutische Formulierung nach Anspruch 1, umfassend:
ein Fusionsprotein zwischen IL-2 und P64k.

6. Therapeutische Formulierung nach Anspruch 1, ferner umfassend:
einen monoklonalen Antikörper für IL-2, einen Krebsimpfstoff auf der Basis von spezifischen Tumorantigenen oder Wachstumsfaktoren und/oder einen monoklonalen Antikörper für CD25.

7. Therapeutische Formulierung nach Anspruch 1,
in Kombination mit einem Krebsimpfstoff, basierend auf spezifischen Tumorantigenen oder Wachstumsfaktoren, zur Verwendung bei der Behandlung von Krebs.

8. Therapeutische Formulierung nach Anspruch 7, wobei der Krebsimpfstoff EGF umfasst.

9. Therapeutische Formulierung nach Anspruch 1,
in Kombination mit einem spezifischen monoklonalen Antikörper für CD25 zur Verwendung bei der Behandlung von Krebs.

10. Therapeutische Formulierung nach Anspruch 1, ferner umfassend:
einen spezifischen monoklonalen Antikörper für CD25.

11. Therapeutische Formulierung nach den Ansprüchen 1 bis 7 zur Herstellung eines Medikamentes zwecks Auslösung einer Immunreaktion auf IL-2, die das Wachstum von Tumoren bei Krebspatienten zu hemmen vermag.

## Revendications

1. Formulation thérapeutique destinée à déclencher une réponse immunitaire contre l'IL-2 en vue de son utilisation dans le traitement de patients cancéreux, comprenant de l'IL-2 humaine recombinante couplée à une protéine transporteuse par conjugaison génétique ou chimique, ladite protéine transporteuse étant la protéine P64k de *Neisseria meningitidis,* et un adjuvant approprié.

2. Formulation thérapeutique destinée à déclencher une réponse immunitaire contre l'IL-2 selon la revendication 1, dans laquelle l'adjuvant est choisi dans le groupe comprenant l'hydroxyde d'aluminium et le Montanide ISA 51.

3. Formulation thérapeutique destinée à déclencher une réponse immunitaire contre l'IL-2 selon la revendication 1, dans laquelle l'adjuvant est le Montanide ISA 51.

4. Formulation thérapeutique destinée à déclencher une réponse immunitaire contre l'IL-2 selon la revendication 1, comprenant une conjugaison chimique entre l'IL-2 et la P64k.

5. Formulation thérapeutique destinée à déclencher une réponse immunitaire contre l'IL-2 selon la revendication 1, comprenant une protéine de fusion entre l'IL-2 et la P64k.

6. Formulation thérapeutique destinée à déclencher une réponse immunitaire contre l'IL-2 selon la revendication 1, comprenant en plus un anticorps monoclonal anti-IL-2, un vaccin anticancéreux à base d'antigènes tumoraux ou de facteurs de croissance spécifiques, et/ou un anticorps monoclonal anti-CD25.

7. Formulation thérapeutique selon la revendication 1 en combinaison avec un vaccin anticancéreux à base d'antigènes tumoraux ou de facteurs de croissance spécifiques, destinée à être utilisée dans le traitement du cancer.

8. Formulation thérapeutique destinée à déclencher une réponse immunitaire contre l'IL-2 selon la revendication 7, dans laquelle le vaccin anticancéreux comprend de l'EGF.

9. Formulation thérapeutique destinée à déclencher une réponse immunitaire contre l'IL-2 selon la revendication 1 en combinaison avec un anticorps monoclonal anti-CD25 spécifique, destinée à être utilisée dans le traitement du cancer.

10. Formulation thérapeutique destinée à déclencher une réponse immunitaire contre l'IL-2 selon la revendication 1, comprenant en plus un anticorps monoclonal anti-CD25 spécifique.

11. Formulation thérapeutique selon les revendications 1 à 7 pour fabriquer un médicament destiné à déclencher une réponse immunitaire contre l'IL-2 et capable d'inhiber la croissance de tumeurs chez des patients cancéreux.
